# FASCICULE DE BREVET EUROPEEN

(11) **EP 1 373 553 B1**
(45) Date de publication et mention de la délivrance du brevet: **13.06.2007**
(21) Numéro de dépôt: 01978020.4
(22) Date de dépôt: 11.10.2001
(51) Int. Cl.: C12Q 1/04, C12Q 1/24, C12Q 1/34

(54) **PROCEDE D'EXTRACTION DE MICRO-ORGANISMES INTACTS A PARTIR DE BOUES OU DE BIOFILMS**
VERFAHREN ZUR GEWINNUNG VON INTAKTEN MIKROORGANISMEN AUS SCHLÄMMEN ODER BIOFILMEN
METHOD FOR EXTRACTING INTACT MICRO-ORGANISMS FROM SLUDGE OR BIOFILMS

(30) Priorité: 11.10.2000 BE 200000648
(43) Date de publication de la demande: 02.01.2004
(73) Titulaire: Université de Liège, 4020 Liège (BE)
(72) Inventeur: HEINEN, Ernst, B-4453 Juprelle (BE); ZORZI, Willy, B-5100 Seraing (BE); ZORZI, Danièle, B-4680 Oupeye (BE); EL MOUALIJ, Benaissa, B-4020 Liège (BE); MELEN, Laurence, B-4920 Harzé (BE)
(74) Mandataire: Colens, Alain M.G.M.
(86) Numéro de dépôt international: PCT/BE2001/000179
(87) Numéro de publication internationale: WO 2002/031184

(56) Documents cités:
- WO-A-01/93875
- DE-A1- 4 421 446
- SALHANI N ET AL: "Improved quantification of aggregated bacteria by combined enzymatic and mechanical treatment of flocs and biofilm from a rotating drum bioreactor" WATER RESEARCH, ELSEVIER SCIENCE PUBLISHERS, AMSTERDAM, NL, vol. 32, no. 4, 1 avril 1998 (1998-04-01), pages 1287-1295, XP004116541 ISSN: 0043-1354
- BOURRAIN MURIEL ET AL: "DNA extraction from activated sludges." CURRENT MICROBIOLOGY, vol. 38, no. 6, juin 1999 (1999-06), pages 315-319, XP002205479 ISSN: 0343-8651
- JORAND F ET AL: "Chemical and structural (2D) linkage between bacteria within activated sludge flocs" WATER RESEARCH, ELSEVIER SCIENCE PUBLISHERS, AMSTERDAM, NL, vol. 29, no. 7, 1 juillet 1995 (1995-07-01), pages 1639-1647, XP004035249 ISSN: 0043-1354 cité dans la demande
- DATABASE WPI Section Ch, Week 198921 Derwent Publications Ltd., London, GB; Class D15, AN 1989-156574 XP002205480 & JP 01 099696 A (KENSETSUSHO DOBOKU), 18 avril 1989 (1989-04-18)

## Description

La présente invention concerne entre autres un procédé de surveillance de consortium de micro-éléments comme les consortiums bactériens -matrice extracellulaire, plus particulièrement ceux que l'on retrouve dans les boues ou les biofilms comme par ex. ceux de station d'épuration d'eaux usées ou de canalisation d'eau de rejet ou de surface.

Pareil procédé peut notamment permettre le suivi de micro-organismes surproducteurs d'enzymes (ou OSE) additionnés aux boues activées en vue de leur bioremédiation. Par ailleurs, ce procédé peut également être appliqué au suivi de micro-organismes qui peuvent être génétiquement modifiés.

Pareil procédé peut aussi être avantageusement appliqué à la préparation d'échantillons permettant l'analyse de bactéries pathogènes et/ou opportunistes et/ou autochtones.

On ne dispose pas actuellement de méthode simple d'extraction de micro-organismes adaptée aux matrices environnementales complexes et aux boues activées, qui respecte l'intégrité cellulaire.

A cette fin, selon un premier aspect de l'invention, on fait appel à la sonication.

### ART ANTERIEUR

L'application de la sonication (ou vibrations ultrasoniques) en vue de lyser des bactéries a déjà été décrite pour le traitement de boues, par exemple dans le brevet US 3.994.780 pour améliorer la digestion anaérobie de biomasses. Dans ce brevet, les parois cellulaires sont détruites et libèrent ainsi des enzymes tels que des amylases, protéases, cellulases qui participent à la remédiation des biomasses. La sonication décrite dans ce document est donc utilisée avec un but et un effet différents de ceux recherchés par la présente invention.

Par ailleurs la publication de brevet japonais J06153989 et de brevet danois DK 19940000188 mentionnent également l'utilisation de la sonication pour désagréger des microbes fixés sur des supports ou pour désagréger les bactéries de boues activées. Enfin, Jorand F. dans Wat. Res., 1995, 29(7), 1639-1647 divulgue l'extraction des polymères extra-cellulaires dans les boues activées par sonication et présente un modèle de structure du floc. La structure des flocs est composée d'organismes microscopiques divers , de micro- et macromolécules, de polymères, de glycoprotéines, de substances hydrophobes, de polysaccharides, de protéines et de composés organiques et inorganiques divers. Cette composition varie en fonction de la nature, de l'âge, et la Provenance des boues (Zita, A. and Hermanson, M., 1994).

Le document DE 4421446A1 divulgue un procédé de traitement de boues industrielles comprenant une huile. Ce procédé comprend une étape de sonication en cascade incorporant plusieurs sources d'ultrasons.

La sonication a également été utilisée pour l'extraction de DNA à partir de boues activées (Bourrain Muriel et al. Current Microbiology, vol. 38, no 6, juin 1999, pages 315-319).

Nazir Salhani et al (Wat. Res. Vol 32 No 4 pp 1287-1295 (1998)) décrivent un procédé d'analyse de consortium de micro éléments biologiques de type bactérien dans lequel un échantillon est soumis à l'action conjuguée, séquentielle, de vibrations ultrasoniques et d'une digestion enzymatique. Un procédé similaire est décrit dans le document japonais JP 01099696 A (Kentsetsusho Doboku).

Il existe cependant toujours un besoin pour une méthode améliorée permettant de convertir un consortium en des unités de formation de colonie (CFU) avec un minimum de destruction.

### BUT DE L'INVENTION

L'invention concerne notamment un procédé de désorganisation de matrices complexes en l'occurrence de flocs de boues activées, libérant les micro-organismes intacts, et qui est compatible avec les méthodes de détection bactériologique classiques ou avec les méthodes de détection d'antigènes telles que l'étalement sur milieux sélectifs, l'hybridation in situ, les techniques d'immunodétection (comme l'ELISA, l'immuno-PCR, ...) la cytométrie en flux, et la spectrométrie de masse (exemples d'application : MALDI-MS-TOF ("Matrix-assisted laser desorption/ionization mass spectrometry including time of flight"), ESI-MS (Electrospray ionization mass spectrometry)).

### RESUME DE L'INVENTION

L'invention propose un procédé d'analyse et d'extraction de consortium de micro-éléments, plus particulièrement du contenu microbien de boues ou de biofilms, selon la revendication 1 annexée dans lequel un échantillon de boue ou de biofilm , éventuellement dilué, est soumis à l'action, de vibrations ultrasoniques et éventuellement d'une digestion enzymatique. La sonication est une sonication fractionnée.

### EXPOSE DETAILLE DE L'INVENTION

L'invention propose notamment un procédé amélioré de surveillance de la qualité microbienne de boues de station d'épuration d'eaux usées dans lequel des échantillons de boue sont soumis à l'action de vibrations ultrasoniques qui, selon un mode de réalisation préféré, est suivi par une digestion enzymatique via un cocktail d'enzymes.

Le mélange d'enzymes est destiné à dégrader les agrégats de biopolymères extra-cellulaires qui stabilisent les flocs présents dans la boue et qui se retrouvent sous forme de suspension dans le surnageant de sonication.

Par la combinaison de la sonication et la digestion enzymatique, la quantité de microorganismes intacts et/ou vivants libérés est, de manière surprenante, bien supérieure non seulement par rapport à la quantité présente dans le surnageant de décantation de la boue non traitée mais aussi par rapport aux quantités détectables par la sonication ou la digestion opérée séparément. Ces traitements se sont avérés avantageusement complémentaires.

La fréquence des vibrations ultrasoniques est de préférence comprise entre 15 et 35 KHz.

Selon un autre aspect de l'invention, la sonication ou ultrasonication est fractionnée, les micro-organismes libérés après chaque cycle de sonication sont en effet soustraits du milieu, ce qui diminue avantageusement leur temps d'exposition aux vibrations ultrasoniques et diminue donc la mortalité. A la connaissance du demandeur cette technique est nouvelle et n'a donc jamais été appliquée à la libération de microorganismes à partir de boues ou de biofilms ou de toutes autres matrices complexes.

En appliquant le procédé de l'invention, on a par exemple constaté que la quantité totale de micro-organismes extraits est de 100 à 2000 fois plus élevée que dans le surnageant de sédimentation de la boue avant traitement et que cette quantité est de 1,4 à 10 fois plus élevée que celle obtenue pour la boue totale non défloculée.

Selon un aspect avantageux de l'invention, le traitement ultrasonique est complété par un traitement avec un cocktail d'enzymes. Les enzymes peuvent être des hydrolases comme par exemple des lipases, amylases, cellulases, protéases, alcalases. Le choix et les quantités relatives de ces enzymes peuvent être adaptées aux buts précis recherchés et aux types de boues ou de biofilms à traiter. Ainsi par exemple, si des protéines (exemples : toxines, prions, marqueurs de bactéries pathogènes) doivent être détectées, on évitera la présence de protéases dans le cocktail enzymatique.

On a constaté que la combinaison de la sonication, fractionnée ou non, et du traitement enzymatique fournit presque toujours un résultat bien supérieur à celui atteint par une de ces méthodes appliquées séparément.

### Domaine d'application du procédé:

Le procédé de l'invention s'applique également et avantageusement aux biofilms.

Les biofilms existent pratiquement à chaque endroit là où des micro-organismes peuvent proliférer à proximité d'un support non toxique.

Les biofilms ont plusieurs finalités:
1. Ils donnent une résistance accrue aux micro-organismes qui les composent, et ceci contre des traitements anti-bactériens tels que les biocides, antibiotiques, prédateurs, anticorps.
2. Ils permettent la mise en commun de matériaux tels que les biopolymères extracellulaires produits par les bactéries. Celles-ci forment des consortiums équivalents à ce que l'on retrouve dans les boues activées au niveau des flocs et des micro-flocs.
3. L'accès à certains nutriments peut être facilité grâce à des associations et des synergies entre les bactéries du microfilm.

La composition biomoléculaire des biofilms est fort proche de celle des flocs et microflocs de boues et comprend des glycoprotéines, des substances hydrophobes, des polysaccharides, des protéines et des substances humiques (Microbiological Aspects of Biofilms and Drinking water; 2000, Steven L. Percival, James T. Walker, Paul R. Hunter ; CRC Press LCC, Boca Raton; London ; Washington,D.C.).

Ces biofilms sont très récalcitrants et , pour être éliminés, exigent des traitements drastiques tels que la sonication ou un traitement enzymatique (voir par exemple documents de brevet WO0153010, WO0123534).

La sonication qu'elle soit fractionnée ou non, combinée à une digestion enzymatique selon la présente invention, permet également d'améliorer la désorganisation des biofilms avec libération (extraction) accrue et/ou maximale des micro-organismes vivants et/ou intacts en vue de leur analyse par des méthodes décrites ci-dessous. Cette application permet également de libérer des agents pathogènes tels que des bactéries , virus, spores, oocystes et autres agents transmissibles non conventionnels comme les protéines prions qui se seraient combinés au biofilm et pour lesquels des techniques d'extraction adéquates font défaut dans l'art antérieur.

Le temps de la digestion enzymatique est extrêmement variable et dépend de l'activité enzymatique ainsi que de la qualité de la boue à défloculer.

Lorsqu'une extraction complète est souhaitée, le fractionnement du traitement par sonication s'arrête quand on ne visualise plus de résidus (flocs) de boue dans le tube à soniquer, le nombre d'étapes ou cycles de la sonication fractionnée dépend ainsi de l'efficacité des étapes successives de traitement de défloculation.

### EXEMPLES

Les exemples suivants illustrent plusieurs aspects de la méthode selon l'invention en se référant aux graphiques annexés. L'évaluation de l'extraction microbienne a été réalisée à l'aide d'un spectromètre par mesure de turbidimétrie à 550 nm et par dénombrement de colonies de bactéries poussant sur milieu de culture gélosé ("Plate Count Agar").

### Activité des enzymes utilisés

a) Enzymes solubles:
   α-amylases
      - Aquazym 120 L:: 120 Kilo Novo (α-amylase Units / g
      = 120 NU / g
      - AMG 300 L:: 300 KNU / g
      (1 KNU = la quantité d'enzyme pour hydrolyser 5,26 g d'amidon a 37°C pH 5,6)
   Lipases
      - Lipolase 100T:: 100 Kilo Lipase Units / g
      - Novozym 868:: 800 KLU / g
   Cellulases
      - Novozym 188L:: 250 Cellobiase Units / g = 250 CbU / g
      - Viscozyme L:: 120 FBG / ml
      - Celluclast 1,5 LFG:: 700 Endo-Glucanase Units/g= 700 EGU/g
      - Denimax 991 L:: 1500 Acid Cellulase Units/g= 1500 ACU/g
   Protéases
      - Savinase 16L:: 16 Kilo Novo Protease Units/g = 16 KNPU/g
      - Neutrase 0,5L:: 0,5 AU / g
      - Flavourzyme 1000L:: 1000 Leucine Amino-Peptidase Units/g = 1000 LAPU /g
      - Alcalase 2,5L :: 2,5 Anson Units /g = 2,5 AU / g
b)Enzymes sur support ou en poudre:
   α-amylases:
      BAN: 800 Kilo Novo (α-amylase Units / g = 800 KNU / g
   Lipases:
      Lipolase 100T : 100 Kilo Lipase Units = 100 KLU
   Cellulases :
      Celluzym 0,7 T : 700 Detergent Cellulase Units/g = 700 DCU / g
   Protéases:
      - Neutrase 1,5 MG:: 1,5 Anson Units / g = 1,5 AU / g
      - Alcalase 2,0T :: 2 Anson Units / g = 2 AU / g
      - Flavourzyme 500 MG:: 500 Leucine Amino-Peptidase Units/g = 500 LAPU /g
      - Savinase 6,0 T:: Kilo Novo Protease Units /g = 6 KNPU

On a utilisé un générateur à ultrasons de la marque LABSONIC auquel est adapté une sonde standard de 19 mm de diamètre de la même marque.

### Boue utilisée :

La boue utilisée provient de la station d'Embourg (Belgique) de capacité de 27000 équivalents habitants fonctionnant de façon homogène sans problèmes majeurs de "foaming" ou de "bulking" (développement de micro-organismes filamenteux formant des enchevêtrements résultant en une écume, la boue décantant très lentement et s'épaississant très peu avec pour conséquence un gonflement et donc une augmentation du volume des boues). Au cours de différents essais, des échantillons de boues différents ont été prélevés à des moments différents de l'année. Des exemples représentatifs sont présentés ci-après.

Les échantillons sont prélevés à la sortie du bassin d'aération. Ils sont maintenus à 4°C pendant environ 24 h avant le processus d'extraction. La conductivité est de façon générale inférieure à 1 mS (Siemens), plus précisément entre 0,7 et 0,8 mS.

### Exemple 1

### Sonication simple

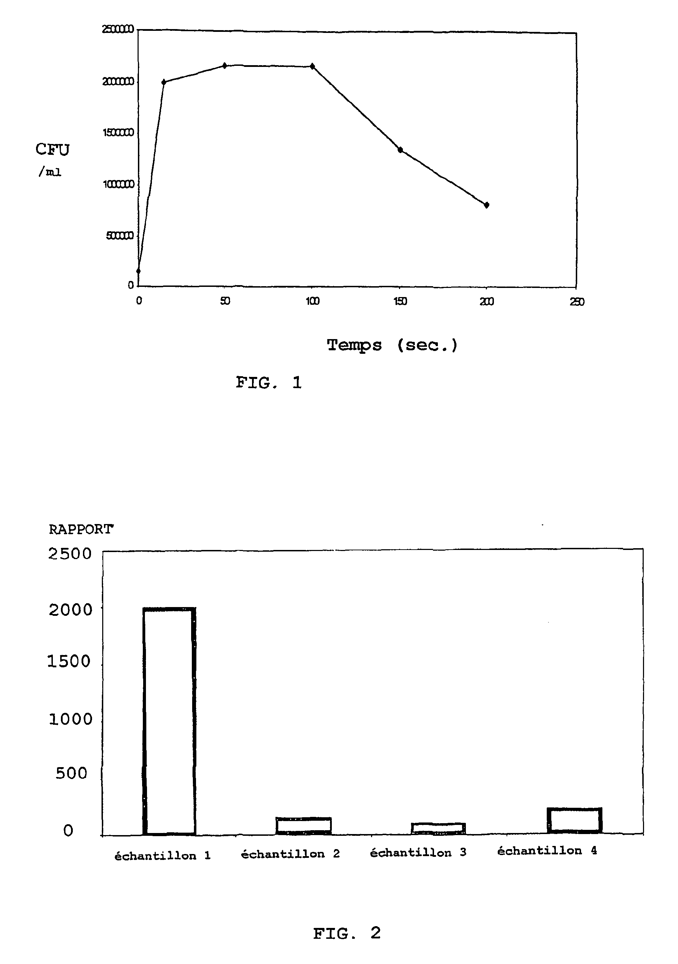

Un échantillon de 40 ml de boue, d'origine susmentionnée, est placé dans un tube conique de 50 ml et incubé sur glace pendant 15 minutes. La boue est ensuite soniquée (37 W), la sonde étant immergée de 15 à 25 mm de profondeur dans le liquide. Une série de temps de sonication a été testée : 15, 50, 100, 150 et 200 secondes. La Fig. 1 illustre l'évolution du nombre de CFU en fonction du temps de sonication, dans le surnageant après sédimentation (15 minutes au repos).

Le CFU correspond à une unité bactérienne formant une colonie. Lors de l'étalement d'une suspension diluée de bactéries vivantes sur un milieu gélosé adéquat, chaque bactérie vivante donnera donc une colonie.

On observe qu'au cours des 15 premières secondes de sonication, l'augmentation du nombre de micro-organismes libérés dans le surnageant est très importante, au moins 13 fois supérieure à la quantité de micro-organismes qu'il y avait au départ dans le surnageant de sédimentation de la boue.

Si l'on observe l'évolution de la densité optique du surnageant à 550 nm, on constate que des particules sont libérées jusqu'à 150 secondes de sonication. Par après, l'absorbance diminue.

Les microflocs peuvent être observés au microscope pendant les premières 50 secondes de sonication. Par après il n'y en a pratiquement plus.

Au delà de 60 secondes de sonication, des tests de viabilité réalisés à partir de cultures pures indiquent une mortalité cellulaire très importante (Jorand F. dans Wat. Res., 1995, 29(7), 1639-1647).

### Exemple 2

### Sonication fractionnée

Un échantillon de 40 ml de boue, d'origine susmentionné, est soniqué pendant 15 secondes sur glace dans les conditions de l'exemple 1. Après sédimentation de la boue résiduelle pendant 15 minutes, le surnageant est extrait. Le restant de la boue est resuspendu dans un tampon d'extraction dont la conductivité est proche de celle de l'échantillon (Na₃PO₄ 2mM, NaH₂PO₄ 4mM, NaCl 9 mM et KCl 1 mM, pH7) de façon à obtenir à nouveau un volume de 40 ml. L'échantillon est traité à nouveau de la même façon, jusqu'à ce qu'il ne reste plus de flocs dans le tube à soniquer, soit après 7 cycles. Les surnageants de sonication sont rassemblés et maintenus sur glace.

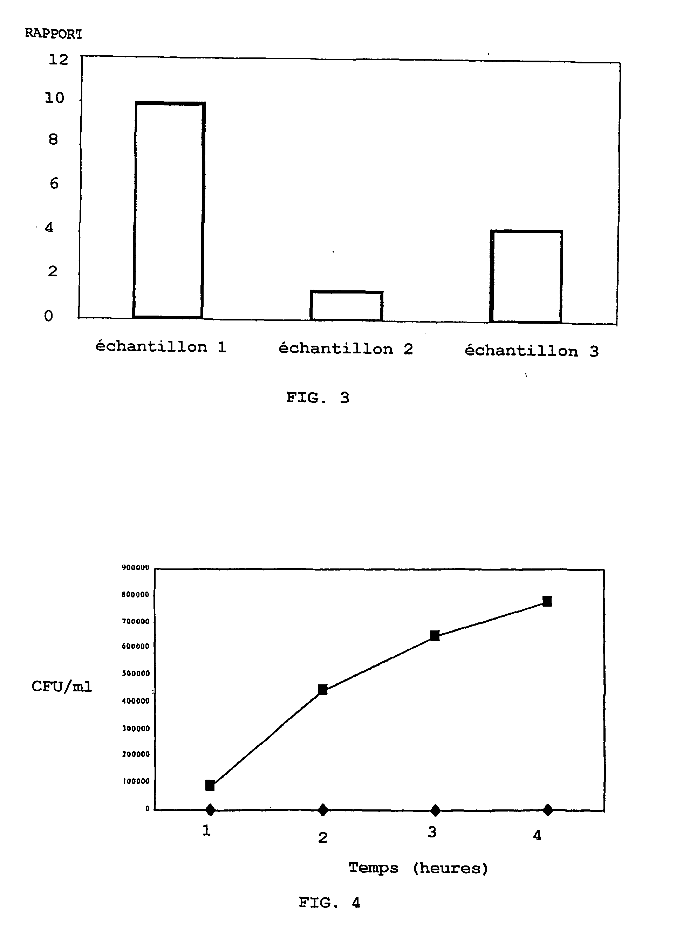

Le graphique de la Fig. 2 indique le rapport observé pour quatre échantillons entre le nombre de CFU/ml obtenus après défloculation par sonication fractionnée et le nombre de CFU dans le surnageant , après une sédimentation de 15 minutes, de la boue avant traitement. Quoique que ce rapport soit extrêmement variable d'un échantillon à l'autre, même de même origine, on constate que la quantité totale de micro-organismes mise en évidence est de 100 à 2000 fois plus élevée dans les surnageants issus de la sonication fractionnée et rassemblés par rapport à la quantité totale de micro-organismes dénombrés à partir du surnageant (après sédimentation)de boue non-traitée (Fig. 2). Cette quantité est de 1,4 à 10 fois plus élevée que celle obtenue lors de l'étalement de la boue non défloculée et non sédimentée sur milieu gélosé (Plate Count Agar), comme indiqué à la Fig. 3 où ce rapport est illustré pour 3 échantillons.

### Exemple 3

### Digestion enzymatique simple

Trois expériences ont été réalisées sur boue totale en ajoutant des quantités variables d'enzymes BAN, Neutrase (qui se présentent sous forme de poudre), Alcalase, Lipolase 100T, Cellulase (qui se trouvent sur un support insoluble), Savinase et Flavourzyme (liquides). Le mélange est remis en suspension et centrifugé ; le surnageant contenant la fraction solubilisée des enzymes est récupéré et constitue ce qu'on appelle, dans les expériences suivantes, la solution de stock. Les billes de support peuvent en effet interférer avec la mesure de turbidimétrie au spectrophotomètre.
1 - Dans la première série d'expériences, 200 ml de boue ont été traités avec une composition comprenant 8 mg de chaque enzyme en granule ou en poudre et 8 µl d'enzyme liquide (solubilisée), soit environ 1% par rapport à la matière sèche, et cela après 1, 2, 3 heures d'incubation.
   Aucun effet n'a été observé quant au nombre de CFU (Colony Forming Unit) dans le surnageant de sédimentation.
2 - Dans une autre série d'expériences, on a utilisé la cellulase à une concentration 100 fois plus élevée, et augmenté de 10 fois les quantités de Lipolase, Alcalase et Neutrase. Les quantités de BAN, Flavourzyme et Savinase sont restées les mêmes que pour la première série d'expériences.
   Comme illustré à la Fig. 4, après une heure d'incubation, le nombre de CFU dans le surnageant de sédimentation a augmenté d'un facteur 5 par rapport à l'échantillon non traité et après 3 heures d'incubation, il a augmenté d'un facteur 9 par rapport à l'échantillon non-traité.
3 - Enfin, toutes les enzymes ont été ajoutées en quantités dix fois supérieures par rapport à la première série d'expériences sur une boue préalablement rincée 2 fois et resuspendue dans le tampon d'extraction. Dans le cas des enzymes sur support, la solution stock est centrifugée et le surnageant est récupéré car les billes de support empêchent la lecture au spectromètre.

Une augmentation d'un facteur 6 du nombre de CFU a été observée après 1 h 30 d'incubation par rapport à l'échantillon non traité. On peut encore observer au microscope la présence de micro-flocs dans le surnageant de sédimentation, prouvant que la défloculation n'est que partielle avec l'utilisation des enzymes seules.

### Exemple 4

### Sonication fractionnée suivie d'une digestion enzymatique

- 40 ml de boue sont soumis à la sonication fractionnée dans les conditions décrites précédemment (exemple 2).
- les 6 fractions résultant de ce traitement , chacune d'un volume inférieur à 40 ml, sont rassemblées; un volume total de 180 ml est ainsi obtenu,
- 40 ml de ce mélange est transféré dans un tube et 16 µl de chacune des enzymes suivantes y sont ajoutées (soit 10% de chaque enzyme par rapport au poids sec de la boue):
   Aquazym 120 l, AMG 300 L, Lipolase, Novozym 868, Novozym 188, Viscozyme L, Celluclast 1,5 LFG, Denimax 991 L, Savinase, Neutrase, Flavourzyme 500 MG, Alcalase

La digestion enzymatique est réalisée à température ambiante avec une agitation pendant une heure.

Lors de cette expérience, la sonication fractionnée a permis d'améliorer d'un facteur 6 la quantité de micro-organismes comptabilisée dans la boue totale avant traitement.

Après la digestion enzymatique, la quantité de micro-organismes est 20 fois supérieure par rapport à la boue totale avant traitement.

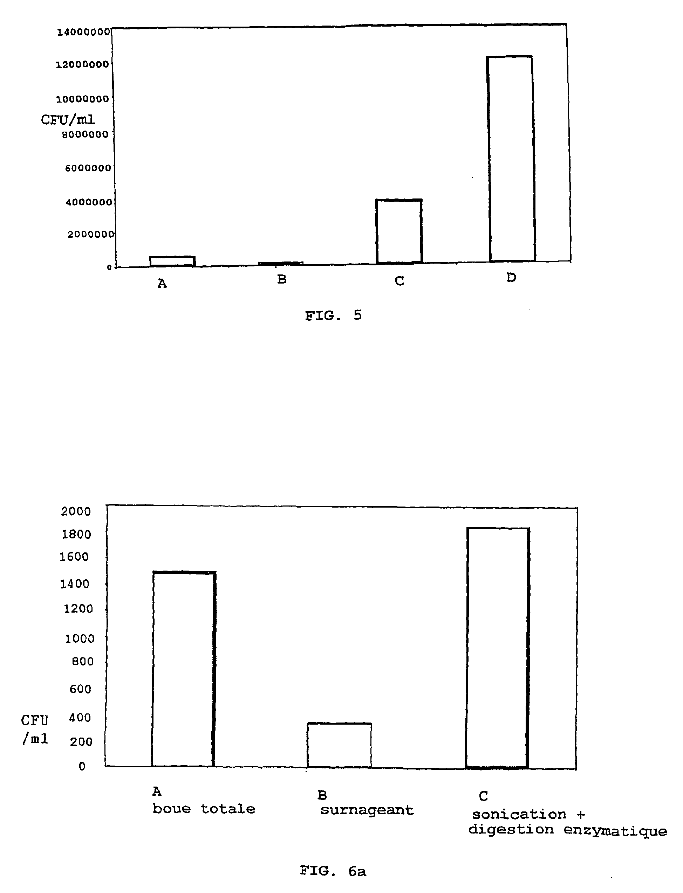

Cet effet est illustré à la Fig. 5 dans laquelle A indique le résultat pour un échantillon de boue totale non traitée et non sédimentée, B indique le résultat pour le surnageant de la boue sédimentée 15 minutes, C indique le résultat après sonication fractionnée et D le résultat après sonication fractionnée et digestion enzymatique.

La composition du mélange d'enzyme peut être modulée en fonction de la matrice de façon à obtenir les meilleurs rendements d'extraction de micro-organismes vivants et les conditions précises de sonication dépendront du type de matrice environnementale ou de boues activées ou autres.

Il est bien entendu que le procédé de l'invention peut être combiné avec d'autres traitements tels que l'ajout d'EDTA, d'EGTA, d'agents chélateurs d'ions multivalents, la filtration ou des gradients de Percoll, etc..

### Exemple 5

Effet de la dilution de l'échantillon de boue sur l'efficacité de la sonication :

Deux tubes contenant 40 ml de boue totale et 40 ml de boue diluée 2 fois en tampon d'extraction ont été soniqués comme dans les conditions habituelles. En tenant compte du facteur de la dilution pour le deuxième échantillon, on constate que la dilution de la boue totale améliore le "rendement" d'extraction de micro-organismes viables: dans notre exemple, une dilution d'un facteur 2 de l'extraction engendre la libération de 2 fois plus de micro-organismes dès la première étape de sonication.

### Exemple 6 :

### Extraction de bactéries pathogènes :

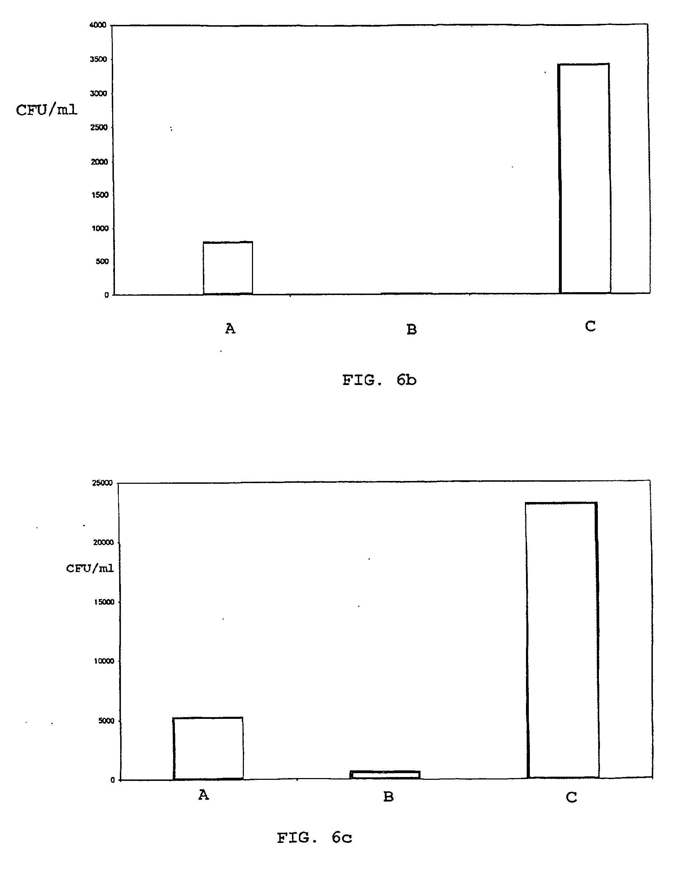

Un échantillon de 40 ml de boue diluée est soniquée 15 secondes sur glace, comme dans l'exemple 1, à une puissance de 37 W. Après digestion pendant 20 minutes avec 144 µl de chaque enzyme du cocktail sous agitation à température ambiante, on constate que la quantité totale de bactéries pathogènes mise en évidence est supérieure par rapport au surnageant de sédimentation (Figs. 6a, b et c). Ce résultat est valable aussi bien pour les *E.coli, Acinetobacter,* coliformes thermotolérants que pour d'autres micro-organismes pathogènes. La Fig. 6a illustre le nombre de CFU/ml pathogènes de E.coli obtenus à partir de la boue non traitée et non sédimentée (A), à partir du surnageant après 15 minutes de sédimentation (B), et après traitement rapide par dilution, suivie de sonication et digestion enzymatique (C). La Fig. 6b montre les mêmes résultats obtenus pour des pathogènes et Acinetobacter, et la Fig. 6c pour des pathogènes cofilormes thermotolérants.

Les bactéries ainsi extraites peuvent être analysées notamment par MALDI MS (phénotypage).

### Exemple 7 :

### Application de l'invention pour l'extraction des protéines:

Le protocole expérimental utilisé est identique à celui utilisé comme pour les autres exemples, en vue d'extraire les bactéries pathogènes et/ou opportunistes et/ou autochtones.

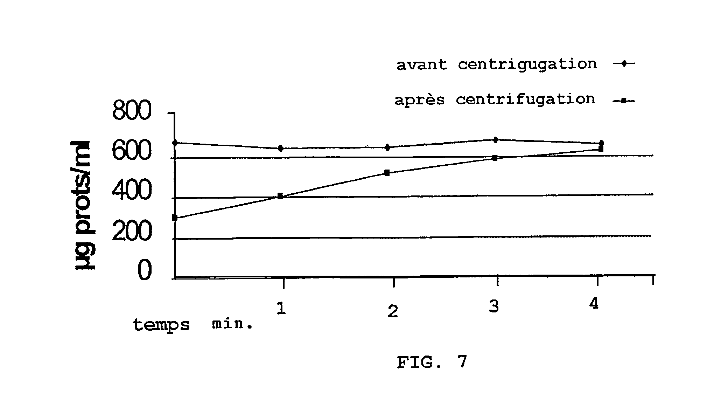

40 ml de boue ont été soniqués à 37 W pendant 1 et 5 minutes en plongeant la sonde à 1,5 cm de profondeur. On laisse sédimenter pendant 15 minutes. Après centrifugation du surnageant 10 minutes à 12000 g et resuspension du culot dans 1 ml d'eau, puis traitement par le cocktail enzymatique de l'exemple 3.3, toutefois sans protéase, on réalise un dosage par le kit Bio-rad Protein Assay de la firme « Bio-rad ». On constate qu'après 4 minutes de sonication, la quasi-totalité du contenu en protéine se retrouve dans le surnageant. Ce protocole modifié a permis de quantifier les protéines et de démontrer que la sonication permet d'extraire les protéines de la boue (Fig. 7) par un procédé compatible avec un dosage ELISA des protéines spécifiques de certaines bactéries comme par exemple celles de Bacillus subtilis.

Etant donné que la sonication fractionnée combinée à des enzymes (en absence de protéases) permet d'améliorer le rendement du procédé d'extraction au niveau des bactéries, il est évident que la libération des protéines dans le surnageant s'en trouve augmentée par rapport au traitement non combiné.

### Exemple 8 :

### Comparaison de la sonication simple et fractionnée :

On prélève un échantillon de 80 ml de boue d'origine susmentionnée. 40 ml de cet échantillon est soumis à une sonication pendant 15 secondes sur glace dans les mêmes conditions que dans l'exemple 1. Après sonication on laisse la boue sédimenter pendant 15 minutes et le surnageant résiduel est prélevé ; les micro-organismes de ce surnageant sont ensuite dénombrés sur « Plate Count Agar ». D'autre part , les 40 ml restants sont soumis à 6 cycles successifs de sonication fractionnée, les surnageants résiduels après chaque cycle de sonication et sédimentation, sont rassemblés (le volume final de ce surnageant rassemblé étant de 260 ml) et on réalise un dénombrement de micro-organismes de ce surnageant final selon le même protocole décrit ci-dessus. On observe qu'au cours des cycles de sonication fractionée le nombre de micro-organismes libérés dans le surnageant est 3 fois supérieur à ce qui a été observé avec la sonication simple.

## Revendications

1. Procédé d'analyse d'un biofilm ou d'une boue **caractérisé en ce qu'**il comprend une étape dans laquelle la boue, éventuellement diluée, ou le biofilm en solution, est traité par un rayonnement ultrasonique, une partie au moins du surnageant étant ensuite soustraite et remplacée par une solution aqueuse, la boue ou le biofilm étant à nouveau traité par un rayonnement ultrasonique, et le surnageant résultant étant combiné avec le surnageant initialement soustrait, les opérations de sonication, soustraction de surnageant et addition de solution pouvant être répétées plus de deux fois, et les surnageants combinés étant soumis à la dite analyse.

2. Procédé selon la revendication 1 dans lequel les temps de sonication varient de 5 à 30 secondes.

3. Procédé selon la revendication 1 dans lequel l'analyse est une analyse bactériologique.

4. Procédé selon la revendication 1 dans lequel, après les cycles de sonication, les surnageants, éventuellement combinés et/ou concentrés, sont soumis à une digestion enzymatique.

5. Procédé d'analyse de consortium de micro-éléments biologiques de type bactérien **caractérisé en ce qu'**il comprend une étape dans laquelle un échantillon est soumis à l'action conjuguée, simultanée ou séquentielle, de vibrations ultrasoniques et d'une digestion enzymatique **caractérisé en ce que** l'ultrasonication est fractionnée en plusieurs cycles, une partie au moins du surnageant étant chaque fois soustraite et remplacée par une solution aqueuse.

6. Procédé selon la revendication 5 dans lequel les échantillons sont des échantillons de boue ou de biofilm, éventuellement dilués, qui sont soumis à l'action de vibrations ultrasoniques, de préférence à une fréquence comprise entre 15 et 35 KHz, puis d'une digestion enzymatique.

7. Procédé selon n'importe laquelle des revendications 5 et 6 dans lequel la digestion enzymatique est opérée sur des suspensions de surnageant séparés des résidus de boues ou de biofilms et résultant de la défloculation obtenue par l'action de vibrations ultrasoniques.

8. Procède selon n'importe laquelle des revendications 5 à 7 dans lequel la digestion enzymatique est effectuée par un mélange d'enzymes contenant des alpha-amylases, des protéases, des cellulases et des lipases.

9. Procédé selon n'importe laquelle des revendications 5 à 8 dans lequel l'ultrasonication fractionnée est menée en plusieurs cycles de durée et de fréquence variable.

10. Procédé d'analyse selon n'importe laquelle des revendications précédentes appliqué à l'extraction de protéines.

## Claims

1. A process for the analysis of a biofilm or of a sludge **characterized in that** it includes a step in which the sludge, possibly diluted, or the biofilm in solution, is treated by an ultrasonic radiation, at least one part of the supernatant being then removed and replaced by an aqueous solution, the sludge or the biofilm being treated again by an ultrasonic radiation and the resulting supernatant being combined with the initially removed supernatant, the steps of the sonication, the removal of the supernatant and the solution additionbeing able to be repeated more than two times and the combined supernatants being subjected to the said analysis.

2. Process according to claim 1 in which the sonication time varies between 5 and 30 seconds.

3. Process according to claim 1 in which the analysis is a bacteriological analysis.

4. Process according to claim 1 in which, after the sonication cycles, the supernatants, possibly combined and/or concentrated, are subjected to an enzymatic digestion.

5. A procees for the analysis of a consortium of biological micro-elements of bacteriological type **characterized in that** it includes a step in which a sample is subjected to a conjugated action, simultaneously or sequentially, of ultrasonic vibrations and of an enzymatic digestion **characterized** such that the ultrasonication is fractionated in several cycles, at least one part of the supernatant being removed each time and replaced by an aqueous solution.

6. Process according to claim 5 in which the samples consist of sludge or biofilm, possibly diluted, that are subjected to the action of ultrasonic vibrations, preferably at a frequency between 15 to 35 KHz, followed by an enzymatic digestion.

7. Process according to either claims 5 and 6 in which the enzymatic digestion is operated on supernatant suspensions separated from sludge or biofilm and resulting from the defloculation obtained by the action of ultrasonic vibrations.

8. Process according to either claims 5 to 7 in which the enzymatic digestion is accomplished by a mixing of enzymes containing alpha-amylases, proteases, cellulases and lipases.

9. Process according to either claims 5 to 8 in which the fractionated ultrasonication is operated in several cycles of variable duration and frequency.

10. Process of analysis according to the previous claims applied to the extraction of proteins.

## Patentansprüche

1. Analyseverfahren von Biofilmen und Schlämmen, dahingehend einen Schritt enthaltend, be idem der Schlamm, eventuell verdünnt, oder der Biofilm in Lösung mit Ultraschall behandelt wird, wobei mindestens eine Teilmenge des Überstandes entnommen und durch eine wässrige Lösung ersetzt wird, der Schlamm oder Biofilm erneut mit Ultraschall behandelt wird und der resultierende Überstand mit dem initial abgenommenen Überstand vereinigt wird, die Ultraschallbehandlungen, Entnahme der Überstände und Beifügen von Lösungen mehr als zweimal wiederholt werden können, und die vereinigten Überstände der genannten Analyse unterzogen werden.

2. Verfahren nach Anspruch 1, wobei die Ultraschallbehandlung zwischen 5 und 30 Sekunden variiert.

3. Verfahren nach Anspruch 1, wobei die Analyse eine bakteriologische Analyse ist.

4. Verfahren nach Anspruch 1, bei der die Zyklen der Ultraschallbehandlung, die Überstände, eventuell kombiniert und/oder konzentriert, einem enzymatischen Verdau unterzogen werden.

5. Analyseverfahren eines Gemenges biologischer Spurenbestandteile bakterieller Art, dahingehend einen Schritt enthaltend, bei dem eine Probe der dualen Anwendung, zeitgleich oder sequentiell, von Ultraschallwellen und eines enzymatischen Verdaus unterzogen wird, dahingehend **gekennzeichnet**, dass die Ultraschallbehandlung in mehrere Zyklen aufgeteilt ist, wobei zumindest eine Teilmenge des Überstandes jeweilig entnommen und durch eine wässrige Lösung ersetzt wird.

6. Verfahren nach Anspruch 5, wobei die Proben Proben eines Schlammes oder Biofilms sind, eventuell verdünnt, die der Wirkung von Ultraschallwellen unterzogen werden , präferentiell mit einer Frequenz zwischen 15 und 35 KHz, gefolgt von einem enzymatischen Verdau.

7. Verfahren nach einem der Ansprüche 5 und 6, wobei der enzymatische Verdau auf µSuspensionen von Überstanden realisiert wird, die van Schlamm- und Biofilmrückständen befreiten und durch die Aktion der Ultraschallwellen entflockt wurden.

8. Verfahren nach einem der Ansprüche 5 bis 7, bei dem der enzymatische Verdau durch ein Enzymgemisch, welches alpha-Amylasen, Proteasen, Zellulasen und Lipasen enthält.

9. Verfahren nach einem der Ansprüche 5 bis 8, bei dem die fraktionierte Ultraschallbehandlung in mehreren Zyklen mit variabler Dauer und Frequenz durchgeführt wird.

10. Analyseverfahren nach einem der vorigen Ansprüche zur Extraktion von Proteinen.
